# FASCICULE DE BREVET EUROPEEN

(11) **EP 1 144 588 B1**
(45) Date de publication et mention de la délivrance du brevet: **09.09.2009**
(21) Numéro de dépôt: 99934783.4
(22) Date de dépôt: 27.07.1999
(51) Int. Cl.: C12N 1/20, C12N 9/28, A21D 8/04

(54) **SOUCHES DE BACTERIES LACTIQUES, LEUR PROCEDE DE CULTURE ET LEURS APPLICATIONS**
MILCHSÄUREBAKTERIEN, DEREN KULTIVIERUNGSVERFAHREN UND VERWENDUNGEN
NOVEL LACTIC ACID BACTERIA, CULTURE METHOD AND USES

(30) Priorité: 28.07.1998 FR 9809652
(43) Date de publication de la demande: 17.10.2001
(73) Titulaire: Institut de Recherche pour le Développement ( IRD), 75480 Paris Cedex 10 (FR)
(72) Inventeur: GUYOT, Jean-Pierre, F-34000 Montpellier (FR); MORLON-GUYOT, Juliette, F-34000 Montpellier (FR); AMPE, Frédéric, F-31000 Toulouse (FR); TALAMOND, Pascale, F-13630 Eyragues (FR); RAIMBAULT, Maurice, F-34980 Saint Clement de Riviere (FR)
(74) Mandataire: Peaucelle, Chantal
(86) Numéro de dépôt international: PCT/FR1999/001847
(87) Numéro de publication internationale: WO 2000/006695

(56) Documents cités:
- US-A- 5 316 776
- OLASUPO N A ET AL: "Studies on local strains of amylolytic Lactobacillus from Nigerian fermented foods." NAHRUNG, vol. 40, no. 1, 1996, pages 45-46, XP002100551
- HOUNHOUIGAN D J ET AL: "Characterization and frequency distribution of species of lactic acid bacteria involved in the processing of mawe, a fermented maize dough from Benin" INTERNATIONAL JOURNAL OF FOOD MICROBIOLOGY, vol. 18, no. 4, 1993, pages 279-287, XP002100552
- AGATI V ET AL: "Isolation and characterization of new amylolytic strains of Lactobacillus fermentum from fermented maize doughs (mawe and ogi) from Benin." JOURNAL OF APPLIED MICROBIOLOGY, vol. 85, no. 3, septembre 1998 (1998-09), pages 512-520, XP002120424
- "DSMZ - Strain DSM 20055 (Lactobacillus cellobiosus)" DSM ONLINE CATALOG, XP002100563 http://www.dsmz.de/strains/no020055.htm

## Description

L'invention a pour objet de nouvelles souches de bactéries lactiques, leur procédé de culture et leurs applications.

Elle vise en particulier des souches de bactéries lactiques utilisables pour l'élaboration de levains en remplacement des couples levures/bactéries lactiques traditionnellement mis en oeuvre, ou pour améliorer la synergie avec les levures habituellement employées.

Les levains utilisés pour la fabrication de pains dits "au levain" à partir de céréales, blé ou seigle, sont généralement constitués d'une association de levures et de bactéries lactiques associées ou non à des enzymes amylolytiques, les α-amylases. Ces enzymes sont endogènes (amylases des céréales) ou exogènes (apport extérieur, amylases fongiques). Dans ces levains, les levures interviennent essentiellement par leur capacité à produire du gaz carbonique qui provoque le gonflement de la pâte. Les bactéries lactiques contribuent aux caractéristiques organoleptiques des pains au levain, à une production complémentaire de gaz carbonique et à une meilleure conservation du pain. Quant aux amylases, en produisant l'hydrolyse de l'amidon, elles permettent d'augmenter la disponibilité en substrat utilisable par les levures et les bactéries.

Dans Nahrung, vol 40, n°1, 1996, p43-46, Olasupo et al rapportent des souches amylolytiques isolées d'aliments fermentés du Niger. Mais ces souches ne produisent pas d'amylase de type alpha.

Le brevet US 5 316 776 se rapporte à une méthode de fermentation de grain moulu et fait référence à des espèces de *Lactobacillus* produisant des isoamylases.

Dans Int. J. of Food Microbiology, vol. 18, n°4, 1993, p279-287, Hounhouigan et al décrivent des souches de bactéries lactiques de produits fermentés. Mais il s'agit de souches qui ne sont pas amylolytiques.

La recherche de bactéries lactiques plus performantes que celles utilisées à ce jour a amené les inventeurs à étudier les souches bactériennes présentes dans les aliments fermentés d'origine céréalière tels que ceux consommés en Afrique. Leurs travaux ont porté plus particulièrement sur les préparations fermentées à base de manioc, ou encore de bouillies de maïs, comme l'ogi ou le mawé consommés au Bénin. L'ogi est formé d'un gruau obtenu à partir d'une suspension de maïs moulu, préparé à partir de grains humides, et le marré est une bouillie acide obtenue à partir de mais écrasé à sec.

A partir de ces produits fermentés, les inventeurs ont réussi à isoler de nouvelles souches de bactéries lactiques présentant des caractéristiques originales permettant de simplifier l'élaboration des levains tout en leur conférant des propriétés de grand intérêt.

L'invention a donc pour but de fourni: de nouvelles souches de bactéries lactiques.

Elle vise également à fournir un procédé de culture de ces souches.

En outre, en mettant à profit les activées détectées chez les nouvelles souches, l'invention vise leur application en particulier en panification pour la production de pains au levain ou de produits similaires.

Les souches de bactéries lactiques de l'invention sont des souches isolées de leur environnement naturel. Elles sont caractérisées en ce qu'il s'agit de souches de *Lactobacillus fermentum* produisant des amylases de type α et qu'au moins 70 % du gène codant pour cette activité est capable de s'hybrider avec la région de l'ADN plasmidique ou génomique codant pour une amylase de l'une au moins des souches de *Lactobacillus fermentum* déposées à la CNCM le 27 mai 1998, respectivement sous les numéros 1-2028 et 1-2029.

La CNCM est la Collection Nationale de Culture de Microorganismes, Institut Pasteur, 28 rue du Docteur Roux, PARIS (FRANCE).

L'hybridation est réalisée de la manière suivante : l'ADN des souches est fixé sur un support solide et soumis à hybridation dans des conditions de forte stringence avec une sonde marquée. Ainsi dans des conditions appropriées, cette sonde peut s'hybrider avec la séquence complémentaire et la localisation s'effectue par autoradiographie. Les conditions d'hybridation correspondent à celles couramment utilisées par l'homme de l'art ; telles que décrites par exemple par Meinkoth et al. (1987) Hybridization of nucleic acids immobilized on solid supports. Anal Biochem. 138, 267-284.

Les souches de bactéries lactiques de l'invention présentent les propriétés de base communes aux bactéries lactiques et sont Gram (+), non-mobiles, catalase (-), et non sporulées.

Il s'agit en particulier de souches se présentant essentiellement sous forme de bâtonnets courts, isolés ou appariés, le cas échéant en chaînes courtes avec occurrence de longs filaments.

Selon une autre disposition de l'invention, les souches bactériennes sont hétérofermentaires et produisent, à partir d'amidon, de l'acide lactique comme acide organique principal, de l'acide acétique, de l'éthanol et du CO₂.

On notera l'intérêt dans de nombreuses applications comme la panification ou la préparation d'aliments fermentés de disposer, grâce à l'invention, de souches capables à la fois d'hydrolyser l'amidon et de produire du CO₂.

Selon encore une autre disposition de l'invention, les souches sont caractérisées en ce que leur croissance s'effectue à un pH de l'ordre de 6,0 à une température de l'ordre de l'ambiante, dans un milieu renfermant de l'amidon.

Le pH optimum de l'activité amylolytique sur substrat d'amidon, évalué sur la base du test de complexation amidon-iode, se situe le plus généralement entre 3,5 et 5,5 à une température de 30 à 45°C environ, le plus souvent de 30-35°C à 45°C.

L'invention vise en particulier les souches de bactéries appartenant à l'espèce *Lactobacillus fermentum,* tel que démontré par les profils SDS-PAGE et les profils de fermentation. On mesurera l'originalité du caractère amylolytique de ces souches de *L. fermentum,* alors que les travaux effectués jusqu'à présent n'avaient jamais permis de détecter une telle propriété, ce qui conduisait à réduire l'importance de ces bactéries lactiques dans les aliments fermentés.

L'invention vise notamment la souche de *Lactobacillus fermentum* déposée à la CNCM le 27 Mai 1998 sous le n° 1-2028. Cette souche comme décrit dans les exemples a été isolée à partir d'ogi.

L'invention vise également la souche de *Lactobacillus fermentum* déposée le 27 mai 1998 à la CNCM sous le n° 1-2029.

Cette souche a été isolée à partir de mawé.

Ces souches sont encore caractérisées en ce que leurs gènes codant pour les amylases ne s'hybrident pas avec les sondes nucléiques spécifiques, disponibles, mises au point pour la détection des gènes d'α-amylases d'autres types de bactéries lactiques homolactiques ou hétérofermentaires facultatives comme *Lactobacillus manihotivorans, Lactobacillus plantarum* A6 ou *Lactobacillus amylovorus.*

De telles sondes nucléiques sont décrites par Giraud E. et al dans Gene 198: 149-158, 1997.

Les souches de *Lactobacillus fermentum* déposées à la CNCM sont en outre caractérisées par des coefficients fermentaires (rapport molaire acide lactique/acide acétique), déterminés en milieu MRS contenant de l'amidon, de l'ordre de 5, et plus précisément de 5,1 pour 1-2028 et de 5 pour 1-2029.

L'α-amylase produite par 1-2028 est fortement active à des pH entre 4,0 et 5,5 et à des températures entre 35 et 45°C pour 1-2028, l'enzyme étant encore active dans les conditions de culture de la souche à une température de 25-30°C et à un pH de 6,0.

L'α-amylase produite par 1-2029 est fortement active entre 3,5 et 5,0 et à des températures entre 30 et 45°C, l'enzyme étant encore active dans les conditions de culture ci-dessus.

Ces deux souches se distinguent également entre elles par les tests suivants :
- tests de l'amidon et du glycogène sur galeries API 50 CHL, qui se révèlent positifs pour la souche 1-2029 et négatifs pour -2028, ces tests étant positifs en milieu MRS,
- utilisation par la souche 1-2029 du L-arabinose : positive, D-mannose : faiblement positive, ces mêmes tests étant négatifs pour 1-2028.

Les mutants des souches définies ci-dessus, et notamment des 2 souches déposées à la CNCM, entrent également dans le cadre de l'invention dès lors qu'ils possèdent une activité amylolytique dans les tests d'évaluation donnés dans les exemples.

Conformément à l'invention, les souches de bactéries lactiques définies ci-dessus peuvent être isolées à partir de préparations fermentées du type ogi ou mawé, sur milieu MRS-agar en utilisant de l'amidon soluble comme substrat, suivi d'une discrimination par RPLF avec comparaison à des souches de *Lb-fermentum* amylolytiques, et de SDS-PAGE, et d'une vérification de leurs propriétés amylolytiques.

La culture des souches de l'invention peut être réalisée dans un milieu de culture tel que ceux utilisés classiquement pour les bactéries, au quel on ajoute de l'amidon soluble en utilisant un inoculum à raison de 5 % (v/v) environ, à une température d'incubation de l'ordre de 25 à 35°C, notamment de 25 à 30°C, pendant 10 à 15 h environ, notamment autour de 12 heures. En fin de fermentation, le pH est de l'ordre de 4 à 5, notamment de 4,3 à 4,5. Ces souches peuvent aussi être cultivées dans les mêmes conditions dans un milieu de culture simplifié (milieur MSA).

On utilise avantageusement comme inoculum des cultures âgées de 10 à 15 h environ, en particulier de 12 h.

L'amidon soluble est ajouté au milieu de culture à raison de 10 à 20 g/l et notamment d'environ 15 g/l.

En variante, les souches de bactéries de l'invention peuvent être cultivées à une température de l'ordre de 25 à 30°C, pendant 10 à 15 h environ, en particulier pendant 12 h, dans une suspension dans l'eau de farine de céréales à raison de 2 à 5 % en poids de farine/volume, avec gélatinisation de la farine. Avantageusement, la croissance des bactéries ne nécessite aucun apport extérieur de facteurs de croissance ou autres compléments. La gélatinisation permet d'améliorer la fermentation de l'amidon.

L'étude des souches de bactéries lactiques de l'invention a permis de mettre en évidence leur activité amylolytique.

Cette activité a été étudiée par
- des tests de caractérisation de halos d'hydrolyse d'amidon autour des colonies en milieux solides sur milieu MRS-agar contenant de l'amidon soluble, et
- par des tests de mise en évidence de l'hydrolyse de l'amidon (méthode de caractérisation du complexe amidon-iode à λ = 620 nm) dans des surnageants de culture en fin de phase exponentielle en milieu MRS-amidon (l'amidon soluble est hydrolysé en moins de 6 heures).

L'utilisation de telles souches ; combinant à la fois les caractères hétérolactique et amylolytique, revêt donc un grand intérêt pour de nombreuses applications concernant en particulier le domaine alimentaire.

De telles souches sont, notamment, tout spécialement appropriées comme starters pour la fabrication de pains au levain à partir de céréales comme le blé ou le seigle ou de produits analogues comme le panetone.

Elles peuvent être utilisées seules, ou bien conjointement avec des levures. Mais l'addition habituelle d'α-amylase n'est plus nécessaire, ce qui simplifie grandement le procédé d'élaboration du levain.

Ces starters peuvent être incorporés lors de l'élaboration du mélange eau/farine de céréales, après gélatinisation si cette opération est mise en oeuvre, et refroidissement pour l'obtention du levain.

Les levains formés sont alors incorporés à la pâte à pain. Les protocoles habituels peuvent être mis en oeuvre, ce qui permet de ne pas alourdir les procédures habituelles et de se conformer aux pratiques des différents pays et fabricants, d'où une grande souplesse d'utilisation. Si un ajout de levure est prévu, il peut être effectué pendant ou après la phase de fermentation lactique.

D'autres caractéristiques et avantages de l'invention seront donnés dans les exemples qui suivent et en se référant aux figures 1 à 6 qui représentent respectivement,
- la figure 1, un dendrogramme phylogénétique montrant les parentés et différences entre les souches 1-2028 et 1-2029 avec d'autres souches de *Lactobacillus fermentum* et des souches de *Lactobacillus curvatus,*
- la figure 2 les cinétiques de dégradation de l'amidon, de croissance et de modification des souches 1-2028 et 1-2029 sur milieu MRS-amidon à 30°C,
- la figure 3, la comparaison de la croissance de la souche 1-2028 sur le milieu MRS et sur le milieu simplifié MSA,
- la figure 4, l'activité extracellulaire amylolytique de la souche 1-2028 en fonction du pH et de la température,
- la figure 5, l'activité extracellulaire amylolytique de la souche 1-2029 en fonction du pH de la température, et
- la figure 6, la détermination de l'activité amylolytique dans le surnageant de culture et liée aux cellules des souches 1-2028 et 1-2029.

### Milieux et conditions de cultures

Pour la culture des souches, on utilise le milieu MRS (de Man et al, Journal of Applied Bacteriology 23, 130-135, 1960), renfermant 2 % d'amidon soluble (MRS-amidon commercialisé par Prolabo).

Ces bactéries peuvent aussi être cultivées en anaérobiose (sous atmosphère d'azote ou de dioxyde de carbone) dans le milieu de culture simplifié (milieu MSA) mis au point à cet effet et dont la composition est la suivante (en g/l) :

| | |
|---|---|
| sulfate de sodium heptahydraté | 0,207 |
| sulfate de manganèse monohydraté | 0,056 |
| monohydrogénophosphate de sodium bihydraté | 3,5 |
| dihydrogénophosphate de potassium anhydre | 6,4 |
| extrait de levure | 20 |
| acétate de sodium anhydre | 5 |

Dans ces conditions, la croissance après 12 h d'incubation est équivalente à celle obtenue en milieu MRS.

Les études de fermentation de l'amidon, sans contrôle du pH, ont été effectuées dans des flacons de type ERLENMEYER de 250 ml à 30°C et en fermenteurs de deux litres. Les isolats ont été maintenus dans du glycérol à 20 %, à -80°C.

### Isolement de bactéries lactiques amylolyliques à partir d'ogi et de mawè:

Le protocole suivant a été utilisé : 10 g d'ogi ou de mawè, préparés dans des petites unités de traitement traditionnelles, ont été échantillonnés, dilués et homogénéisés dans 90 ml de solution stérile de peptone.

Des dilutions décimales ont été étalées sur des plaques d'agar- (MRS-amidon + bleu d'aniline 1 %).

Après 3 jours d'incubation à 30°C, on observe des colonies colorées en bleu avec le bleu d'aniline, avec des zones d'hydrolyse de l'amidon révélées par des vapeurs d'iode.

### . Caractérisation des isolats

12 souches amylolytiques ont été isolées et purifiées par trois transferts successifs sur des plaques de MRS-amidon-agar.

Ces colonies font de 1 à 2 mm de diamètre. Elles apparaissent circulaires, convexes, opaques et blanches.

En exposant les plaques à des vapeurs d'iode, on met en évidence tout autour des halos de 1 à 1,5 cm de diamètre.

On note la présence d'amylase dans le milieu avec toutes les souches.

### . Caractères phénotypiques et biochimiques

La morphologie cellulaire a été observée avec un microscope à contraste de phase.

Le caractère Gram a été déterminé en utilisant un set de coloration Gram-color (Merck KgaA, Darmstadt, Allemagne) et par la méthode au KOH de Gregersen (European Journal of Applied Microbiology and Biotechnology 5,123-127, 1978).

L'activité catalase a été testée comme décrit par Combet-Blanc et al (International Journal of Systematic Bacteriology 45,9-16, 1995).

Le système API 50 CHL a été utilisé pour déterminer la production d'acides à partir des hydrates de carbone (Biomérieux, France).

L'acide lactique, l'acide acétique et l'éthanol dans le surnageant ont été analysés par HPLC, en utilisant une colonne Aminex HPX 87H (BioRad, Richmond, VA, USA) relié à un détecteur d'indice de réfraction (PU 4026 Philips, Heindoven) avec 6 mM d'acide sulfurique comme éluant à une vitesse d'élution de 0,8 ml min ⁻¹ à 65°C.

Les isomères d'acide lactique D,L, ont été déterminés en utilisant une combinaison de tests enzymatiques du commerce (Boehringer Mannheim, Allemagne).

Les résultats de ces études montrent que :
- Les cellules apparaissent Gram-positives, non mobiles, catalase-négatives et non sporulées. Elles se présentent sous forme de bâtonnets courts, isolés, appariés ou parfois sous forme de chaînes courtes. On peut observer parfois de longs filaments.
- Toutes les souches produisent à partir d'amidon, de l'acide lactique comme acide organique principal (mélange d'acide lactique D et L, où D (-) constitue la forme majoritaire), de l'acide acétique, de l'éthanol et du CO₂.

Les schémas de fermentation typique sont indiqués, pour les 2 souches, dans le tableau 1 suivant.

**Tableau 1 : Produits de fermentation des souches 1-2028 et 1-2029**

| Souches | % des formes D/L de l'acide lactique | Acide lactique (g/l) | Acide acétique (g/l) | Ethanol (g/l) |
|---|---|---|---|---|
| I-2028 | 54/46 | 8,78 | 1,15 | 1,94 |
| I-2029 | 53/47 | 8,9 | 1,18 | 2,34 |

Dans le tableau 2, on rapporte les profils de fermentation sur galeries API 50CHL des 2 souches isolées I-2028 et I-2029 (F = faible).

**Tableau 2**

| Hydrates de carbone | I-2028 | I-2029 |
|---|---|---|
| Témoin | - | - |
| Glycérol | - | - |
| Erythritol | - | - |
| D-Arabinose | - | - |
| L-Arabinose | - | + |
| Ribose | - | + |
| D-Xylose | - | + |
| L-Xylose | - | - |
| Adonitol | - | - |
| β-Méthyl-D-Xyloside | - | - |
| Galactose | + | + |
| D-Glucose | + | + |
| D-Fructose | +F | + |
| D-Mannose | - | +/- |
| L-Sorbose | - | - |
| Rhamnose | - | - |
| Dulcitol | - | - |
| Inositol | - | - |
| Mannitol | - | - |
| Sorbitol | - | - |
| α-Méthyl-D-Mannoside | - | - |
| α-Méthyl-D-Glucoside | - | - |
| N-Acétyl-Glucosamine | - | - |
| Amygdaline | - | - |
| Arbutine | - | - |
| Esculine | - | - |
| Salicine | - | - |
| Cellobiose | - | - |
| Maltose | - | + |
| Lactose | + | +/- |
| Mellibiose | + | + |
| Sucrose | - | - |
| Trénalose | - | - |
| Inuline | - | - |
| Mélézitose | - | - |
| D-Raffinose | + | + |
| Amidon | - | + |
| Glycogène | - | + |
| Xylitol | - | - |
| ß-Gentobiose | - | - |
| D-Turanose | - | - |
| D-Lyxose | - | - |
| D-Tagatose | - | - |
| D-Fucose | - | - |
| L-Fucose | - | - |
| D-Arabitol | - | - |
| L-Arabitol | - | - |
| Gluconate | +F | +F |
| 2-Céto-gluconate | - | - |
| 5-Céto-gluconate | +F | +F |

Comme le montrent les résultats de ce tableau, les souches, au vu de leurs profils de fermentation sont différentes.

Les souches fermentent les sucres suivants : L-arabinose, galactose, D-glucose, maltose, mellibiose, sucrose et D-raffinose ; le gluconate et le 5-céto-gluconate ne sont que faiblement fermentés.

Le D-fructose montre une réaction positive faible pour 1-2028 par rapport à la réaction positive forte obtenue avec I-2029.

1-2028 fermente le D-xylose et le lactose, alors que I-2029 fermente le ribose, l'amidon, le glycogène et faiblement le D-mannose et le lactose.

Des essais répétés avec 1-2028 donnent toujours des réactions négatives lorsqu'on utilise l'amidon et le glycogène. Le système API 50 CHL ne permet donc pas toujours l'identification de bactéries lactiques fermentant l'amidon, même si cette fermentation est démontrée dans les milieux solides ou liquides MRS-amidon.

### ETUDE DES SOUCHES I-2028 et I-2029

Les souches 1-2028 et 1-2029 déposées à la CNCM ont été choisies comme souches représentatives de chaque groupe.

### Analyses SDS PAGE et des clusters.

On prépare des extraits protéiques de cellules entières et on réalise la SDS-PAGE comme décrit par Pot et al (Chemical methods in Bacterial Systematics. Godfellow, M. and O'Donnel (eds). J. Willey and Sons, Chichester, 1994).
La méthode de Pot et al, et le système de software GelCompar (version 4.0), (Vauterin et al, European Journal of Microbiology 1, 37-41, 1992) sont utilisés pour enregistrer les motifs d'électrophorèse des protéines, normaliser les tracés de densitométrie, grouper les souches (utilisation du coefficient de corrélation du moment du produit de Pearson (Proceedings of the National Academy of Sciences USA. 85, 2444, 2448, 1988) et analyser les clusters de groupes pairs non pesés en utilisant une liaison moyenne.

Les profils protéiques des souches I-2028 et I-2029 ont été comparés avec une base de données constituée d'empreintes de protéines normalisées de souches de références appartenant à pratiquement toutes les espèces déjà décrites de bactéries lactiques (Pot et al,. The Lactic Acid Bacteria (Chapter 14 ; pages 81-87). Ed. E.L. Foo, H.G. Griffin, R. Mollby and C.G. Heden. Proceedings of the First Lactic Computer Conférence. Norfolk: Horizon Scientific Press, 1993).

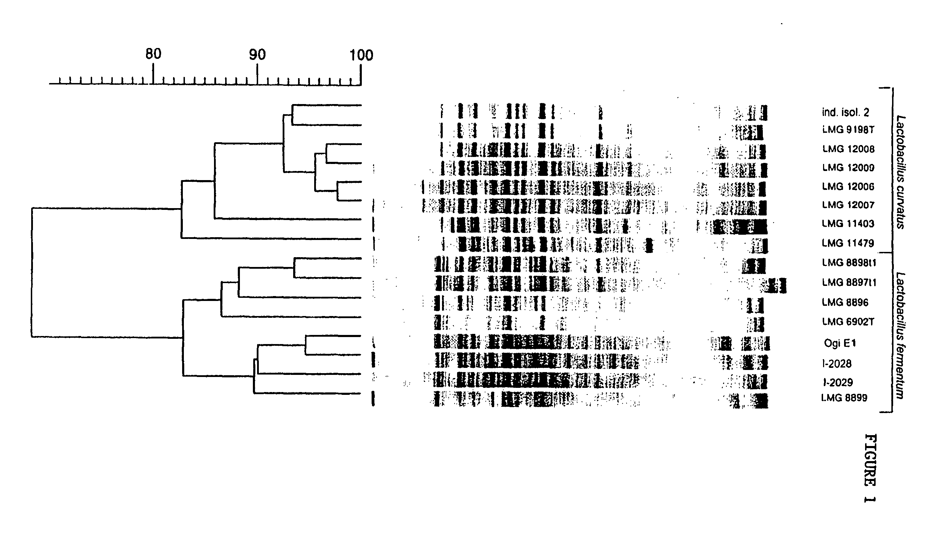

L'analyse numérique des profils protéiques SDS PAGE, représentée comme un dendrogramme, et comprenant des souches représentatives d'espèces significatives des points de vue phénotypique et phylogénétique est donnée sur la figure 1.

L'analyse des clusters des profils protéiques obtenus par SDS PAGE, confirme bien l'appartenance des souches à l'espèce *Lactobacillus fermentum.*

### . Etude de la croissance des bactéries

La croissance des bactéries a été évaluée en mesurant la densité optique à λ = 600nm.

L'acide lactique, l'acide acétique et l'éthanol dans le surnageant ont été analysés par HPLC, en utilisant une colonne Aminex HPX 87H (BioRad, Richmond, VA, USA) relié à un détecteur d'indice de réfraction (PU 4026 Philips, Heindoven) avec 6 mM d'acide sulfurique comme éluant à une vitesse d'élution de 0,8 ml min ⁻¹ à 65°C.

### Etude de l'activité amylase

L'activité amylase a été déterminée en mesurant la capacité de complexation de l'iode par l'amidon comme décrit par Giraud et al cité ci-dessus.

L'activité enzymatique a été déterminée à différents pH (3,5 à 6,5 ; 0,1 mol 1⁻¹ de tampon de citrate-phophate) et températures (30°C à 60°C). L'activité extra-cellulaire a été mesurée sur le surnageant de la culture centrifugée. Pour détecter l'activité amylase liée aux cellules, une culture de 24 heures a été récoltée par centrifugation (5000 rpm, 10 min), le culot cellulaire lavé est mis en suspension dans un sérum physiologique tamponné.

### . Etude de la croissance et de l'acidification à partir d'amidon

On opère en bioréacteurs de 2 litres sans régulation de pH.

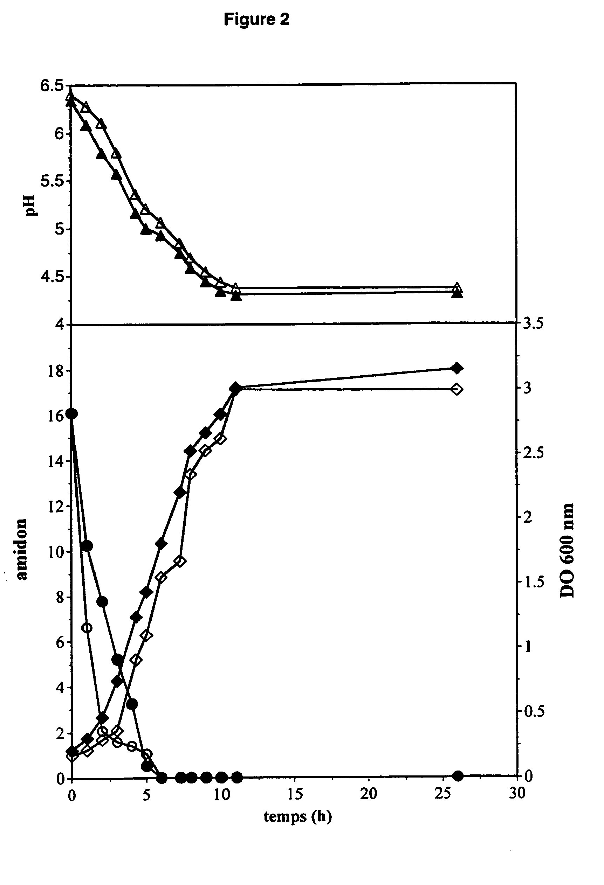

Les résultats obtenus sont donnés sur la figure 2 où les symboles présentent les significations suivantes : pour 1-2028 : amidon (o) ; pH (a) ; DO (◇), et pour 1-2029 : amidon (•) ; pH (▲) ; DO (◆).

On constate que les deux souches poussent en 11 heures sur un milieu liquide MRS contenant de l'amidon (17 g 1⁻¹), à des vitesses de croissance spécifiques de 0,39 et 0,42 h⁻¹, respectivement pour 1-2028 et 1-2029.

L'amidon est hydrolysé rapidement durant les premières heures de la fermentation à une vitesse supérieure avec la souche 1-2028 (7 g 1⁻¹, h⁻¹) par rapport à la souche 1-2029 - (3g, 1⁻¹), h⁻¹).

A la fin de la croissance, les deux souches acidifient le milieu à pH 4,3.

Les vitesses d'acidification sont similaires avec les deux souches : 2,43 et 2,48 unités de pH h⁻¹, respectivement pour 1-2028 et 1-2029.

A la fin de la croissance, les souches produisent la même quantité d'acides organiques et d'éthanol à partir d'amidon, l'acide lactique étant le produit final majoritaire comme le montrent les résultats du tableau 1.

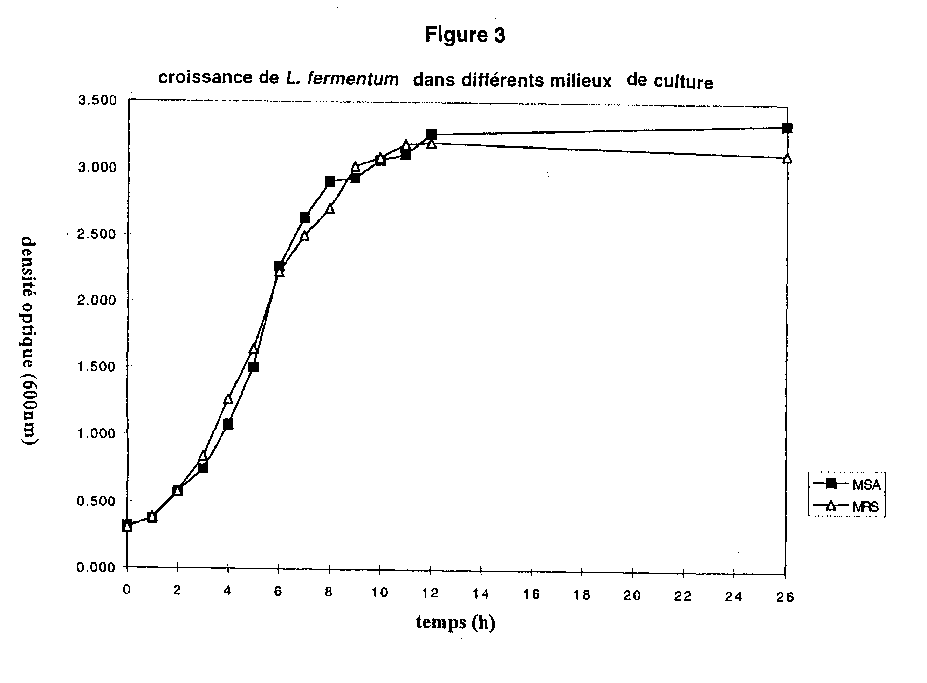

La comparaison de la croissance de la souche 1-2028 sur le milieu MRS et sur le milieu simplifié MSA est montrée à la figure 3.

Etude de l'effet du pH et de la température sur l'activité amylase

On a mesuré les activités amylase extra-cellulaires de surnageants de cultures en phase stationnaire des souches 1-2028 et 1-2029, à différents pH (entre pH 3,0 et 6,5) et températures (entre 30 et 50°C).

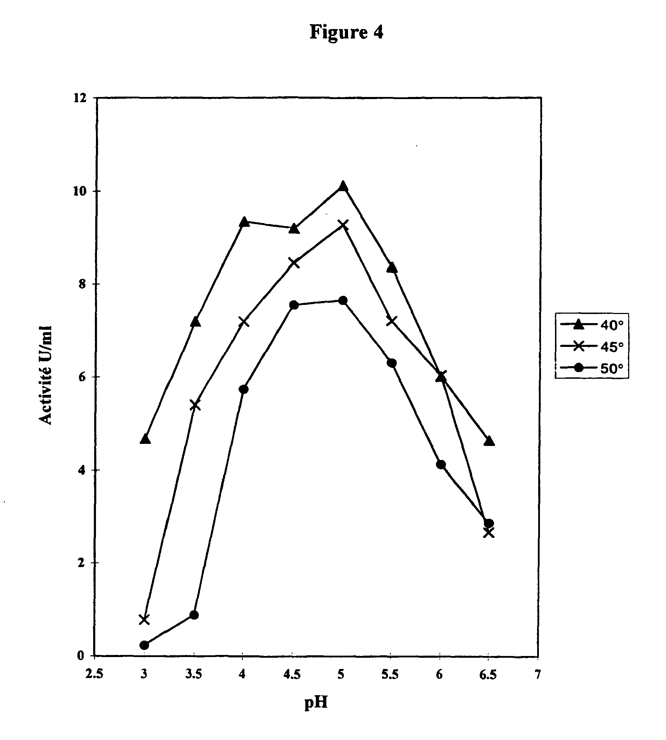

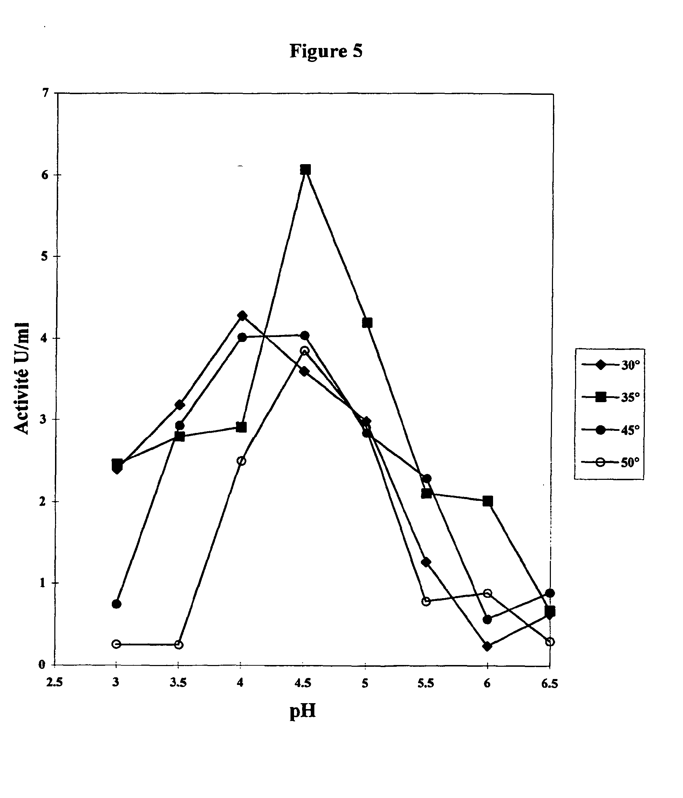

Les résultats obtenus sont donnés sur les figures 4 et 5. On constate que les activités amylase maximales sont obtenues à pH 5,0 et à 40°C, avec la souche 1-2028, et à pH 4,5 et 35°C avec la souche 1-2029.

Dans ces conditions, l'activité amylase extra-cellulaire de la souche 1-2028 est supérieure (10 U ml⁻¹) à celle de la souche 1-2029 (6 U ml⁻¹).

Pour la souche 1-2028 , l'activité amylase extra-cellulaire varie de 8 à 10 U ml⁻¹ à des pH entre 4,0 et 5,5 et des températures entre 35 et 45°C (figure 4).

Pour la souche 1-2029, l'activité amylase extra-cellulaire varie de 4 à 6 U ml⁻¹ à des pH entre 3,5 et 4,5 et des températures entre 30°C et 45°C et décroit rapidement à des pH supérieurs à 5,0 (figure 5).

Les activités amylases liées aux cellules et extra-cellulaires produites par les isolats ont été mesurées et comparées.

Les tests ont été effectués à 45 °C, pH 4,0 et les résultats sont montrés sur la figure 5.

1-2028 produit à la fin de la croissance autant d'activité extra-cellulaire que d'activité amylase liée à la cellule.

Pour 1-2029, les activités liées aux cellules sont beaucoup plus importantes que celles extra-cellulaires.

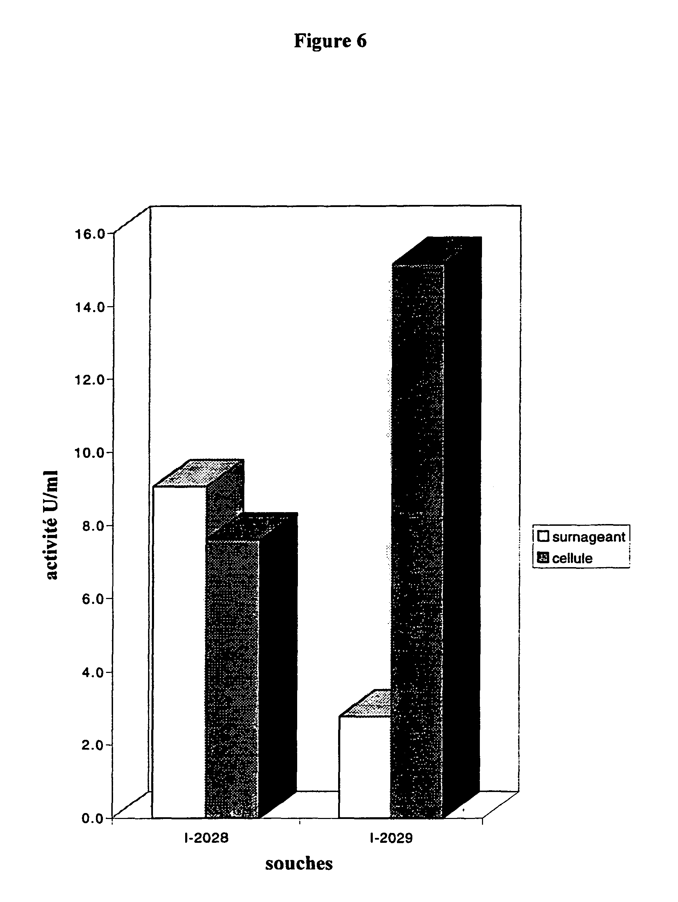

En outre, en comparant les souches I-2028 et 1-2029, on constate que la souche 1-2029 produit moins d'amylase extra cellulaire (2,8 U ml⁻¹ que la souche 1-2028 (9,0 U ml⁻¹), mais produit plus d'amylase liée à la cellule (15,1 U ml⁻¹) que la souche 1-2028 (7,6 U ml⁻¹) (figure 6).

## Revendications

1. Souches de bactéries lactiques isolées de leur environnement naturel, **caractérisées en ce qu'**il s'agit des souches de *Lactobacillus fermentum* déposées à la CNCM le 27 mai 1998, respectivement sous les numéros 1-2028 et 1-2029, et leurs mutants, ces souches et ces mutants produisant des amylases de type α.

2. Souches de bactéries lactiques selon la revendication 1, **caractérisées en ce qu'**elles se présentent essentiellement sous forme de bâtonnets courts, isolés ou appariés, le cas échéant en chaînes courtes avec occurrence de longs filaments.

3. Souches de bactéries lactiques selon la revendication 1 ou 2, **caractérisées en ce qu'**elles sont hétérofermentaires et produisent, à partir d'amidon, de l'acide lactique comme acide organique principal, de l'acide acétique, de l'éthanol et du CO₂.

4. Souches de bactéries lactiques selon l'une quelconque des revendications 1 à 3, **caractérisées en ce que** leur croissance s'effectue à un pH de l'ordre de 6,0 à une température de l'ordre de l'ambiante, dans un milieu renfermant de l'amidon.

5. Souches de bactéries lactiques selon l'une quelconque des revendications 1 à 4, **caractérisées par** un pH optimum d'activité amylolytique entre 3,5 et 5,5, à 30 à 45°C.

6. Procédé de culture des souches selon l'une quelconque des revendications 1 à 5, **caractérisé en ce qu'**on, utilise un milieu de culture tel que ceux utilisés classiquement pour les bactéries, au quel on ajoute de l'amidon soluble en utilisant un inoculum à raison de 5 % (v/v) environ, à une température d'incubation de 25 à 35°C, notamment de 25 à 30°C, pendant 10 à 15 h environ, notamment autour de 12 heures ou qu'en variante, on opère à une température de l'ordre de 25 à 30°C, pendant 10 à 15 h environ, en particulier pendant 12 h, dans une suspension dans l'eau de farine de céréales à raison de 2 à 5 % en poids de farine/volume, avec ou sans gélatinisation de la farine.

7. Utilisation des souches selon l'une quelconque des revendications 1 à 5 comme starters pour la fabrication de levains en panification.

## Claims

1. Strains of lactic acid bacteria isolated from their natural environment, **characterized in that** they are strains of *Lactobacillus fermentum* deposited at the CNCM on 27th May 1998 under numbers I-2028 and 1-2029 respectively, and their mutants, said strains and mutants producing amylases of the α type.

2. Strains of lactic acid bacteria according to claim 1, **characterized in that** they are essentially in the form of short rods, isolated or paired, optionally in short chains with the occurrence of long filaments.

3. Strains of lactic acid bacteria according to claim 1 or 2, **characterized in that** they are heterofermentative and produce, from starch, lactic acid as the main organic acid, acetic acid, ethanol and CO₂.

4. Strains of lactic acid bacteria according to any one of claims 1 to 3, **characterized in that** their growth takes place at a pH of the order of 6.0 at ambient temperature in a medium comprising starch.

5. Strains of lactic acid bacteria according to any one of claims 1 to 4, **characterized by** an optimum pH of amylolytic activity of between 3.5 and 5.5 at 30 to 45°C deposited on 27th May 1998 at the CNCM under no. 1-2028 and 1-2029, and mutants thereof,

6. Process for culture of the strains according to any one of claims 1 to 5, **characterized in that** a culture medium such as those conventionally used for bacteria, to which soluble starch is added, using an inoculum in an amount of about 5% (v/v), is used at an incubation temperature of the order of 25 to 35°C, in particular 25 to 30°C, for about 10 to 15 h, particularly of about 15 h or, as a variant, culturing is carried out at a temperature of about 25 to 30°C, for about 10 to 15 h, in particularly for 12 h, in an aqueous suspension of cereal flour in an amount of 2 to 5% by weight of flour/volume, with or without gelatinization of the flour.

7. Use of strains according to any one of claims 1 to 5 as starters for the production of leavens in panification.

## Patentansprüche

1. Stämme von Milchsäurebakterien, die aus ihrer natürlichen Umgebung isoliert wurden, **dadurch gekennzeichnet, dass** es sich um Stämme von *Lactobacillus fermentum* handelt, die am 27. Mai 1998 unter den Nummern 1-2028 und 1-2029 bei der Collection Nationale de Cultures de Microorganismes (CNCM) hinterlegt wurden, und deren Mutanten, wobei diese Stämme und Mutanten Amylasen des Typs α produzieren.

2. Stämme von Milchsäurebakterien gemäß Anspruch 1, **dadurch gekennzeichnet, dass** sie im Wesentlichen in Form von kurzen Stäbchen, isoliert oder paarweise, gegebenenfalls in kurzen Ketten, vorliegen, wobei lange Filamente auftreten.

3. Stämme von Milchsäurebakterien gemäß Anspruch 1 oder 2, **dadurch gekennzeichnet, dass** sie zur heterofermentativen Milchsäuregärung befähigt sind und aus Stärke Milchsäure als hauptsächliche organische Säure, Essigsäure, Ethanol und CO₂ produzieren.

4. Stämme von Milchsäurebakterien gemäß einem der Ansprüche 1 bis 3, **dadurch gekennzeichnet, dass** ihr Wachstum bei einem pH-Wert in der Größenordnung von 6,0 bei einer Temperatur in der Größenordnung der Raumtemperatur in einem stärkehaltigen Medium stattfindet.

5. Stämme von Milchsäurebakterien gemäß einem der Ansprüche 1 bis 4, **gekennzeichnet durch** ein pH-Optimum der amylolytischen Aktivität zwischen 3,5 und 5,5 bei 30 bis 45 °C.

6. Verfahren zur Kultur der Stämme gemäß einem der Ansprüche 1 bis 5, **dadurch gekennzeichnet, dass** man ein Kulturmedium verwendet, wie man sie klassischerweise für Bakterien verwendet, wozu man lösliche Stärke gibt, wobei man ein Inokulum in einer Menge von ungefähr 5 Vol.-% verwendet, bei einer Inkubationstemperatur von 25 bis 35 °C, insbesondere 25 bis 30 °C, während ungefähr 10 bis 15 Stunden, insbesondere etwa 12 Stunden, oder als Variante arbeitet man bei einer Temperatur in der Größenordnung von 25 bis 30 °C während ungefähr 10 bis 15 Stunden, insbesondere während 12 Stunden, in einer Suspension von Getreidemehl in Wasser in einer Menge von 2 bis 5 Gew.-% Mehl/Volumen, mit oder ohne Verkleisterung des Mehls.

7. Verwendung der Stämme gemäß einem der Ansprüche 1 bis 5 als Starter für die Herstellung von Sauerteigen für die Brotherstellung.
